# EUROPEAN PATENT SPECIFICATION

(11) **EP 1 701 751 B1**
(45) Date of publication and mention of the grant of the patent: **21.12.2011**
(21) Application number: 04811030.8
(22) Date of filing: 15.11.2004
(51) Int. Cl.: A61B 17/04, A61B 17/10, A61B 17/08, A61B 17/00

(54) **IMPROVED FERRULE**
VERBESSERTE ZWINGE
FERRULE AMELIOREE

(30) Priority: 14.11.2003 US 520236 P
(43) Date of publication of application: 20.09.2006
(73) Proprietor: Interventional Therapies, LLC, Westport, Connecticut 06880-3212 (US)
(72) Inventor: SHIKHMAN, Oleg, Trumbull, CT 06611 (US)
(74) Representative: Tripoz, Inès
(86) International application number: PCT/US2004/038144
(87) International publication number: WO 2005/049112

(56) References cited:
- EP-A1- 0 669 101
- WO-A1-00/61012
- WO-A2-01/66001
- FR-A- 494 960
- US-A- 4 470 415
- US-A- 5 752 965
- US-A- 5 879 371
- US-A1- 2003 171 778
- US-B2- 6 720 516

## Description

### BACKGROUND

The present disclosure relates to an instrument and a method for closing a hole or puncture in a blood vessel. More particularly, this disclosure relates to an improved ferrule closure for a hole or puncture in a blood vessel.

When performing catheterization procedures, such an angiography or angioplasty, a catheter is generally introduced into the vascular system by first penetrating the skin, underlying muscle tissue and blood vessel with a sharpened hollow needle. Next, a guide wire is commonly inserted through the lumen of the hollow needle and is caused to enter the selected blood vessel. Subsequently, the needle is typically slid off the guide wire and a combination of a dilator and an introducer (or an introducer alone) are fed over the guide wire and pushed through the skin to enter the vessel. The guide wire can then be removed and the desired catheter to carry out the procedure is fed through the lumen of the introducer and advanced through the vascular system until the working end of the catheter is appropriately positioned. Following the conclusion of the catheterization procedure, the working catheter will be withdrawn and, subsequently, the dilator and/or introducer will also be removed from the wound.

At this point in the procedure, the vessel leakage must be controlled in order to stem the flow of blood through the puncture. Because it is common practice to administer a blood thinning agent to the patient prior to many of the catheterization procedures, stemming the blood flow can be troublesome. A common method of sealing the wound is to maintain external pressure over the vessel until the puncture naturally seals. This method of puncture closure typically takes about thirty minutes, with the length of time usually being greater if the patient is hypertensive or anti-coagulated. In some anti-coagulated patients, the introducer is left in place for hours to allow the anti-coagulant to wear off. When human hand pressure is utilized, it can be uncomfortable for the patient and can use costly professional time on the part of the hospital staff. Other pressure techniques, such as pressure bandages, sandbags or clamps, have been employed, but these devices also require the patient to remain motionless for an extended period of time and the patient must be closely monitored to ensure their effectiveness.
FR 494960 discloses a ferrule according to the preamble of claim 1.

Suture securing instruments are being developed to assist in finalizing the suturing procedure. The presently described ferrule finds application in such suture securing instruments, among others.

### SUMMARY

The improved ferrule includes a biocompatible body portion, the body portion having an aperture therethrough, the body portion also having a first flared region and includes a biocompatible cover material provided on at least a portion of the biocompatible body portion. In one embodiment, the biocompatible body portion is a stainless steel material, a titanium material or the like. In another embodiment, the biocompatible cover material is an expanded polytetrafluoroethylene material or the like. In another embodiment, the biocompatible cover material is a compliant material. In another embodiment, the biocompatible body portion is in a cylindrical form, having an aperture therethrough. In another embodiment, the aperture is configured to receive at least on suture strand, and the biocompatible body portion is deformable to secure the suture strand within the aperture. In another embodiment, the biocompatible cover material is provided at least partially within the aperture of the biocompatible body portion. In another embodiment, the biocompatible body portion has a first end portion and a second end portion, and the second end portion is flared outwardly from the cylindrical body portion. In another embodiment, the biocompatible cover material surrounds the outward flare of the cylindrical body portion.

The above-discussed and other features and advantages of the present invention will be appreciated and understood by those skilled in the art from the following detailed description and drawings.

### BRIEF DESCRIPTION OF THE DRAWINGS

Referring to the FIGURES wherein like elements are numbered alike in the several FIGURES:

FIG. 1 shows an cross sectional view of a first embodiment;

FIG. 2 shows a cross sectional view of a second embodiment;

FIG. 3 shows a cross sectional view of an embodiment positioned at the tip of a suture securing device; and

FIG. 4 shows a cross sectional view of another embodiment positioned at the tip of a suture securing device.

### DETAILED DESCRIPTION OF THE INVENTION

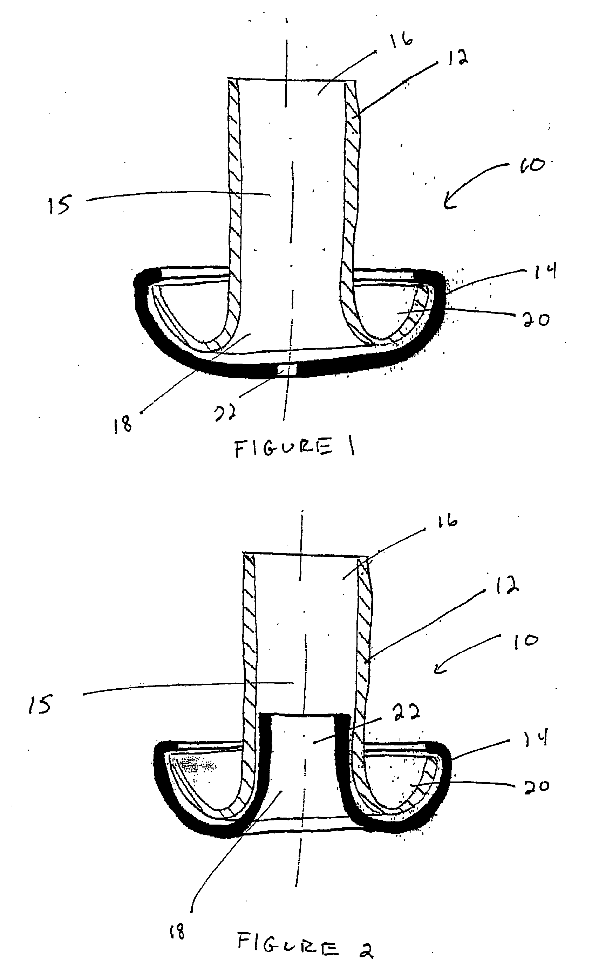

Referring to FIG. 1, an exemplary ferrule 10 is illustrated, comprising a biocompatible body portion 12 and a biocompatible cover portion 14 provided on a portion thereof. An aperture 15 is provided through a portion of the body portion 12. In the illustrated exemplary embodiment, the body portion 12 has a first end portion 16 and a second end portion 18, and the second end portion 18 includes a flared region 20 relative to the first end portion 16. The flared region 20 may be included where, e.g., it is desirable for facile loading of suture or other material therethrough or where it is desirable to facilitate movement of the ferrule through some medium (e.g., down a tissue tract). The flared region 20 may also be provided where it is desirable for the second end portion 18 to have a larger cross sectional width than the first end portion 16. The term "flared" should be construed to include any portion that extends outwardly from a portion of the body portion, whether it be the first end portion 16, part of the second end portion 18 or any region in-between. Furthermore, the flared region 20 may extend outwardly at any angle, including a ninety degree angle, from the remainder of the body portion 12.

Referring still to FIG. 1, the illustrated exemplary embodiment includes cover material 14 provided on the second end portion 18. More specifically, the illustrated exemplary embodiment illustrates the cover material 14 as encircling the flared region 20. Also as illustrated by the exemplary embodiment, the cover material may include a slit or other aperture 22 near the aperture 15 of the body portion 12.

Referring now to FIG. 2, a second exemplary embodiment is illustrated. In this exemplary embodiment, the cover material 14 is provided at least partially within the aperture 15 of the body portion. As in the above described exemplary embodiment, the cover material 14 includes an aperture 22.

The biocompatible body portion may be non-bioabsorbable or bioabsorbable. In one embodiment, the body portion is non-bioabsorbable, such as a stainless steel material, a titanium material or the like. In another embodiment, the body portion is bioabsorbable, e.g., manufactured from a bioabsorbable polymer, such as a homopolymer, copolymer or a blend obtained from one or more monomers selected from the group consisting of glycolic acid, lactide, lactic acid, p-dioxane, E-caprolactone and trimethylene carbonate.

The biocompatible cover material may also be non-bioabsorbable or bioabsorbable. In one exemplary embodiment, the biocompatible compliant material is an expanded polytetrafluoroethylene material, a Teflon material, or the like. In another embodiment, the biocompatible cover material is compliant, such that it will at least conform around adjacent material (e.g., around suture secured within the body portion. The cover material may be secured to the body portion by any convenient mechanism, e.g. by bonding, gluing, shrinking, forming around the body, by elastic properties of the cover material, etc.

In another embodiment, the aperture of the body portion is configured to receive at least one suture strand, and at least a portion of the body portion is deformable to secure the suture strand within the aperture. In such embodiment, the body portion may be deformed by any convenient device or method, e.g. as that described by U.S. Patent No. 5,643,289 to Sauer, , or co-pending United States Patent Publication US2003/078601.

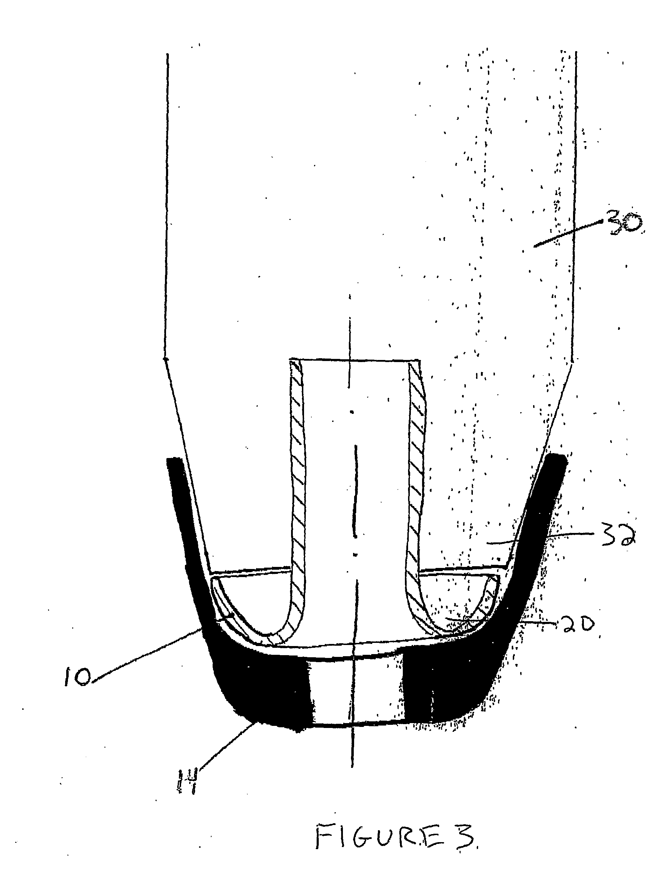

Referring now to the exemplary embodiment illustrated at FIG. 3, an exemplary ferrule 10 is illustrated as loaded onto a positioning and/or crimping device 30. Covering material 14 is provided along portions of the flared region 20. As illustrated, covering material 14 also extends onto a first end portion 32 of the positioning and/or crimping device 30, for example, to facilitate insertion of the device 30 into position in the wound of a patient.

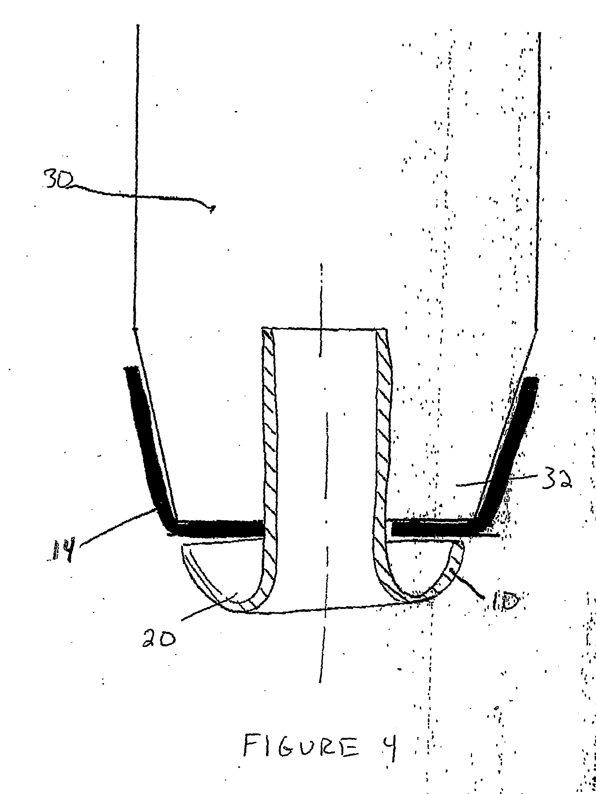

Referring now the exemplary embodiment illustrated at FIG. 4, an exemplary ferrule 10 is illustrated as loaded onto a positioning and/or crimping device 30. Covering material 14 is provided adjacent (on the backside) of portions of the flared region 20. As illustrated, covering material 14 also extends onto a first end portion 32 of the positioning and/or crimping device 30, for example, to facilitate insertion of the device 30 into position in the wound of a patient. In use, the covering material 14 may remain on the device 30 after securing of the ferrule 10 at the wound. Alternately, the covering material 14 may remain with the ferrule 10 and serve (as with the rest of the described embodiments having covering material 14) as a covering material for the portions of the flared region 20 or generally as a pledget for the wound after securing the ferrule 10 at the wound.

While the invention has been described with reference to exemplary embodiments, it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment disclosed as the best mode contemplated for carrying out this invention, but that the invention will include all embodiments falling within the scope of the appended claims.

## Claims

1. A biocompatible ferrule (10), comprising:
a biocompatible body portion (12) having a first end portion (18), a second end portion (16) and a first flared region (20) provided on the first end portion (18), and **characterized in that**
a cover material (14) provided on said first flared region (20) which completely covers said first flared region (20) and serves as a pledget for the wound after securing the ferrule at the wound.

2. The biocompatible ferrule of claim 1, wherein the first flared region (20) extends gradually outwardly from the longitudinal axis of the body portion at increasing angles.

3. The biocompatible ferrule of claim 2, wherein the first flared region (20) extends up to about 45 degrees from the longitudinal axis of the body portion (12).

4. The biocompatible ferrule of claim 3, wherein the first flared region (20) extends up to about 90 degrees from the longitudinal axis of the body portion (12).

5. The biocompatible ferrule of claim 4, wherein the first flared region (20) extends past 90 degrees such that it changes direction with regard to the longitudinal axis of the body portion (12).

6. The biocompatible ferrule of claim 4, wherein the first flared region (20) extends past 90 degrees such that it changes direction with regard to the longitudinal axis of the body portion (12), and wherein the flared region (20) continues to gradually extend outwardly up to about 140 degrees.

7. The biocompatible ferrule of claim 1, wherein such flare is measured solely with reference to the outer surface of the body portion (12).

8. The biocompatible ferrule of claim 1, wherein such flare additionally expands the inner diameter of the body portion (12) where such flare occurs.

9. The biocompatible ferrule of claim 1, wherein said cover material (14) includes an aperture near an aperture of the body portion (12).

10. The biocompatible ferrule of claim 1, wherein said cover material (14) is provided at least partially within an aperture of the body portion (12).

11. The biocompatible ferrule of claim 1, wherein said cover material (14) is non-bioabsorbable.

12. The biocompatible ferrule of claim 11, wherein said cover material (14) is stainless steel or titanium.

13. The biocompatible ferrule of claim 1, wherein said cover material (14) is bioabsorbable.

14. The biocompatible ferrule of claim 13, wherein said cover material (14) is a homopolymer, copolymer or a blend obtained from one or more monomers selected from the group consisting of glycolic acid, lactide, lactic acid, p-dioxane, E-caprolactone and trimethylene carbonate.

15. The biocompatible ferrule of claim 1, wherein said cover material (14) is an expanded polytetrafluoroethylene material or a Teflon material.

16. The biocompatible ferrule of claim 1, wherein said cover material (14) is compliant, such that it will at least conform around adjacent material

17. The biocompatible ferrule of claim 1, wherein said cover material (14) is secured to the body portion by bonding, gluing, shrinking, forming around the body or by elastic properties of the cover material.

18. The biocompatible ferrule of claim 1, wherein an aperture of the body portion is configured to receive at least one suture strand, and at least a portion of the body portion (12) is deformable to secure the suture strand within the aperture.

## Patentansprüche

1. Biokompatible Zwinge (10), umfassend:
einen biokompatiblen Körperabschnitt (12), der einen ersten Endabschnitt (18), einen zweiten Endabschnitt (16) und einen ersten aufgeweiteten Bereich (20), der auf dem ersten Endabschnitt (18) bereitgestellt wird, aufweist, und
**dadurch gekennzeichnet, dass** ein Abdeckmaterial (14) auf dem ersten aufgeweiteten Bereich (20) bereitgestellt wird, das den ersten aufgeweiteten Bereich (20) ganz abdeckt und als Tupfer für die Wunde dient, nachdem die Zwinge an der Wunde befestigt wurde.

2. Biokompatible Zwinge nach Anspruch 1, wobei der erste aufgeweitete Bereich (20) sich von der Längsachse des Körperabschnitts aus unter zunehmenden Winkeln allmählich nach außen erstreckt.

3. Biokompatible Zwinge nach Anspruch 2, wobei sich der erste aufgeweitete Bereich (20) bis ungefähr 45 Grad zur Längsachse des Körperabschnitts (12) erstreckt.

4. Biokompatible Zwinge nach Anspruch 3, wobei sich der erste aufgeweitete Bereich (20) bis ungefähr 90 Grad zur Längsachse des Körperabschnitts (12) erstreckt.

5. Biokompatible Zwinge nach Anspruch 4, wobei sich der erste aufgeweitete Bereich (20) über 90 Grad hinaus erstreckt, so dass er im Verhältnis zur Längsachse des Körperabschnitts (12) die Richtung ändert.

6. Biokompatible Zwinge nach Anspruch 4, wobei sich der erste aufgeweitete Bereich (20) über 90 Grad hinaus erstreckt, so dass er im Verhältnis zur Längsachse des Körperabschnitts (12) die Richtung ändert, und wobei sich der aufgeweitete Bereich (20) bis zu ungefähr 140 Grad weiter allmählich nach außen erstreckt.

7. Biokompatible Zwinge nach Anspruch 1, wobei diese Aufweitung nur mit Bezug auf die äußere Oberfläche des Körperabschnitts (12) gemessen wird.

8. Biokompatible Zwinge nach Anspruch 1, wobei diese Aufweitung zudem den Innendurchmesser des Körperabschnitts (12) an der Stelle erweitert, wo diese Aufweitung erfolgt.

9. Biokompatible Zwinge nach Anspruch 1 , wobei das Abdeckmaterial (14) eine Öffnung neben einer Öffnung des Körperabschnitts (12) umfasst.

10. Biokompatible Zwinge nach Anspruch 1 , wobei das Abdeckmaterial (14) mindestens teilweise in einer Öffnung des Körperabschnitts (12) bereitgestellt wird.

11. Biokompatible Zwinge nach Anspruch 1, wobei das Abdeckmaterial (14) nicht bioabsorbierbar ist.

12. Biokompatible Zwinge nach Anspruch 11, wobei das Abdeckmaterial (14) rostfreier Stahl oder Titan ist.

13. Biokompatible Zwinge nach Anspruch 1, wobei das Abdeckmaterial (14) bioabsorbierbar ist.

14. Biokompatible Zwinge nach Anspruch 13, wobei das Abdeckmaterial (14) ein Homopolymer, ein Copolymer oder eine Mischung ist, die aus einem oder mehreren Monomeren erzielt wird, die aus der Gruppe ausgewählt werden, die aus Gluconsäure, Lactid, Milchsäure, p-Dioxan, E-Caprolacton und Trimethylen-Carbonat besteht.

15. Biokompatible Zwinge nach Anspruch 1 , wobei das Abdeckmaterial (14) ein aufgeschäumtes Polytetrafluorethylen-Material oder ein Teflon-Material ist.

16. Biokompatible Zwinge nach Anspruch 1 , wobei das Abdeckmaterial (14) biegsam ist, so dass es sich mindestens an das angrenzende Material anpasst.

17. Biokompatible Zwinge nach Anspruch 1 , wobei das Abdeckmaterial (14) in dem Körperabschnitt durch Haften, Kleben, Aufschrumpfen, Formen um den Körper oder durch elastische Eigenschaften des Abdeckmaterials gesichert ist.

18. Biokompatible Zwinge nach Anspruch 1, wobei eine Öffnung des Körperabschnitts konfiguriert ist, um mindestens einen Nahtfaden aufzunehmen, und mindestens ein Abschnitt des Körperabschnitts (12) verformbar ist, um den Nahtfaden im Innern der Öffnung zu sichern.

## Revendications

1. Ferrule biocompatible (10), comprenant:
une partie de corps biocompatible (12) présentant une première partie d'extrémité (18), une deuxième partie d'extrémité (16) et une première région évasée (20) prévue sur la première partie d'extrémité (18), et
**caractérisée en ce qu'**un matériau de recouvrement (14) est prévu sur ladite première région évasée (20), qui couvre entièrement ladite première région évasée (20) et sert de tampon pour la blessure après que la ferrule a été fixée à la blessure.

2. Ferrule biocompatible selon la revendication 1, dans laquelle la première région évasée (20) s'étend progressivement vers l'extérieur à partir de l'axe longitudinal de la partie de corps sous des angles croissants.

3. Ferrule biocompatible selon la revendication 2, dans laquelle la première région évasée (20) s'étend jusqu'à environ 45 degrés par rapport à l'axe longitudinal de la partie de corps (12).

4. Ferrule biocompatible selon la revendication 3, dans laquelle la première région évasée (20) s'étend jusqu'à environ 90 degrés par rapport à l'axe longitudinal de la partie de corps (12).

5. Ferrule biocompatible selon la revendication 4, dans laquelle la première région évasée (20) s'étend au-delà de 90 degrés de sorte qu'elle change de sens par rapport à l'axe longitudinal de la partie de corps (12).

6. Ferrule biocompatible selon la revendication 4, dans laquelle la première région évasée (20) s'étend au-delà de 90 degrés de telle sorte qu'elle change de sens par rapport à l'axe longitudinal de la partie de corps (12), et dans laquelle la région évasée (20) continue à s'étendre progressivement vers l'extérieur jusqu'à environ 140 degrés.

7. Ferrule biocompatible selon la revendication 1, dans laquelle cet évasement est mesuré uniquement en se référant à la surface extérieure de la partie de corps (12).

8. Ferrule biocompatible selon la revendication 1, dans laquelle cet évasement dilate de plus le diamètre intérieur de la partie de corps (12) à l'endroit où intervient cet évasement.

9. Ferrule biocompatible selon la revendication 1, dans laquelle ledit matériau de recouvrement (14) comprend une ouverture près d'une ouverture de la partie de corps (12).

10. Ferrule biocompatible selon la revendication 1, dans laquelle ledit matériau de recouvrement (14) est prévu au moins en partie à l'intérieur d'une ouverture de la partie de corps (12).

11. Ferrule biocompatible selon la revendication 1, dans laquelle ledit matériau de recouvrement (14) n'est pas bioabsorbable.

12. Ferrule biocompatible selon la revendication 11, dans laquelle ledit matériau de recouvrement (14) est de l'acier inoxydable ou du titane.

13. Ferrule biocompatible selon la revendication 1, dans laquelle ledit matériau de recouvrement (14) est bioabsorbable.

14. Ferrule biocompatible selon la revendication 13, dans laquelle ledit matériau de recouvrement (14) est un homopolymère, un copolymère ou un mélange obtenu à partir d'un ou de plusieurs monomères sélectionnés dans le groupe composé de l'acide glycolique, du lactide, de l'acide lactique, du p-dioxane, de l'E-caprolactone et du carbonate de triméthylène.

15. Ferrule biocompatible selon la revendication 1, dans laquelle ledit matériau de recouvrement (14) est un matériau de polytétrafluoréthylène expansé ou un matériau de Téflon.

16. Ferrule biocompatible selon la revendication 1, dans laquelle ledit matériau de recouvrement (14) est souple de sorte qu'il s'adapte au moins autour du matériau adjacent.

17. Ferrule biocompatible selon la revendication 1, dans laquelle ledit matériau de recouvrement (14) est ancré à la partie de corps par adhérence, collage, retrait, façonnage autour du corps ou par des propriétés élastiques du matériau de recouvrement.

18. Ferrule biocompatible selon la revendication 1, dans laquelle une ouverture de la partie de corps est configurée pour recevoir au moins un fil de suture, et au moins une partie de la partie de corps (12) est déformable pour ancrer le fil de suture à l'intérieur de l'ouverture.
